# EUROPÄISCHE PATENTSCHRIFT

(11) **EP 1 148 615 B1**
(45) Veröffentlichungstag und Bekanntmachung des Hinweises auf die Patenterteilung: **31.08.2016**
(21) Anmeldenummer: 00119193.1
(22) Anmeldetag: 05.09.2000
(51) Int. Cl.: A61N 1/378, H02J 7/02

(54) **Implantierbare Energiespeicheranordnung für ein medizinisches Implantat sowie Betriebsverfahren dafür**
Implantable energy storage system for a medical implant and operating method therefor
Dispositif implantable d'accumulation d'énergie pour un implant medical et son procédé de fonctionnement

(30) Priorität: 03.04.2000 DE 10016519
(43) Veröffentlichungstag der Anmeldung: 24.10.2001
(73) Patentinhaber: Cochlear Limited, Lane Cove, NSW 2066 (AU)
(72) Erfinder: Wuzik, Martin, 85737 Ismaning (DE); Leysieffer, Hans, 82024 Taufkirchen (DE)
(74) Vertreter: Grünecker Patent- und Rechtsanwälte PartG mbB

(56) Entgegenhaltungen:
- EP-A- 0 480 648
- EP-A- 0 987 808
- US-A- 5 279 292
- US-A- 5 330 515
- US-A- 5 876 423
- WIEGAND M: "ANALYTISCHE BEDINGUNGEN FUER DAS ANSCHWINGEN VON MOS-PIERCE-OSZILLATOREN ANALYTICAL CONDITIONS FOR OSCILLATION START-UP IN MOS PIERCE-OSCILLATORS" FREQUENZ, SCHIELE UND SCHON GMBH. BERLIN, DE, Bd. 39, Nr. 9, März 1985 (1985-03), Seiten 238-242, XP001073597 ISSN: 0016-1136
- PATENT ABSTRACTS OF JAPAN vol. 013, no. 467 (P-948), 23. Oktober 1989 (1989-10-23) & JP 01 183715 A (FUJITSU LTD), 21. Juli 1989 (1989-07-21)

## Beschreibung

Die vorliegende Erfindung betrifft eine implantierbare Energiespeicheranordnung für ein medizinisches Implantat sowie ein Verfahren zum Betreiben einer solchen Energiespeicheranordnung gemäß dem Oberbegriff von Anspruch 1 bzw. 21.

Solche gattungsgemäßen Energiespeicheranordnungen bzw. Verfahren sind beispielsweise aus EP-A-0 987 808, US -A-5,411,537, US-A-5,702,431 sowie US-A 5,713,939 bekannt. Üblicherweise erfolgt das Nachladen von implantierbaren Energiespeicheranordnungen transkutan über eine induktive Strecke mittels eines externen Ladegeräts. Der Ladevorgang wird dabei üblicherweise dadurch gesteuert, dass der Ladestrom und die Spannung des Speichers von einer Steuereinheit gemessen und in entsprechende Steuerimpulse für einen Schalter im Ladestromkreis umgesetzt werden, wobei ein geeignetes Ladeprogramm verwendet wird.

Beim Betrieb des Energiespeichers können zwei unerwünschte Betriebszustände auftreten: Es kann einerseits eine Überladung der Batterie auftreten, wenn der Ladevorgang nicht rechtzeitig beendet wird, was zu Gasentwicklung mit nachfolgender Zerstörung des Speichers führen kann. Andererseits kann die Speicherspannung, wenn ein rechtzeitiges Aufladen des Speichers unterbleibt, auf Werte fallen, die unterhalb der minimalen Betriebsspannung liegen, welche für eine definierte Funktion oder gegebenenfalls zur eingeschränkten Funktion des von dem Energiespeicher zu versorgenden Implantats erforderlich ist. Unter Umständen kann in letzterem Fall die Speicherspannung so weit abfallen, dass auch eine ausreichende Spannungsversorgung der implantatseitigen Elektronik zum Steuern des Ladevorgangs nicht mehr gewährleistet ist. Häufig umfasst die Steuerelektronik ein Mikroprozessorsystem, bei welchem es im Unterspannungsbereich zur Ausführung von falschen logischen Operationen oder zum Verlust der Inhalts von flüchtigen Speichern kommen kann. Somit ist im Unterspannungsbereich bei solchen Systemen eine ordnungsgemäße Ladesteuerung nicht mehr gewährleistet, was dazu führen kann, dass beim Eintritt in den Unterspannungsbereich durch eine Mikroprozessor-Fehlfunktion der Ladepfad hochohmig geschaltet wird und ein Aufladen des Speichers auf diese Weise dauerhaft unterbunden wird.

Selbst wenn eine solche Blockade des Ladepfads nicht eintritt, kann sich ein anderes Problem beim Wiederaufladen des Speichers ergeben. Dieses resultiert aus der Tatsache, dass in stromsparenden Schaltungen häufig Pierce-Oszillatoren eingesetzt werden, welche für ihr erschwertes Anlaufen bekannt sind. Um ein sicheres Einschwingen des Oszillators zu ermöglichen, muss die Versorgungsspannung aus dem Unterspannungsbereich möglichst schnell ansteigen. Beim Aufladen eines wiederaufladbaren Speichers steigt jedoch die Spannung relativ langsam an, was dazu führen kann, dass der Oszillator unter Umständen nicht anlaufen kann, was die Wiederinbetriebnahme des Mikroprozessors verhindert.

Es ist Aufgabe der vorliegenden Erfindung, eine implantierbare Energiespeicheranordnung für ein medizinisches Implantat sowie ein Verfahren zum Betrieb derselben zu schaffen, wobei jederzeit ein sicherer Betrieb gewährleistet ist und insbesondere ein sicheres Wiederaufladen des Energiespeichers auch dann möglich ist, wenn der Speicher so weit entladen wurde, dass die Speicherspannung unter den Normalspannungsbereich für die Steuereinheit abgefallen war.

Diese Aufgabe wird erfindungsgemäß gelöst durch eine implantierbare Energiespeicheranordnung gemäß Anspruch 1 sowie ein entsprechendes Betriebsverfahren gemäß Anspruch 21. Bei dieser erfindungsgemäßen Lösung ist vorteilhaft, dass durch das Vorsehen einer von der Steuereinheit unabhängigen Überwachungseinheit auch bei Ausfall der Steuereinheit, z.B. aufgrund sehr niedriger Speicherspannung bei weit fortgeschrittener Entladung des Speichers, eine ordnungsgemäße Ladekontrolle möglich ist, wobei insbesondere die Wiederaufladbarkeit des Speichers sichergestellt werden kann, indem eine Blockade des Ladepfads wegen einer unterspannungsbedingten Fehlfunktion der Steuereinheit verhindert wird.

In bevorzugter Ausgestaltung der Erfindung steuert die Steuereinheit den Ladevorgang über einen steuerbaren Widerstand im Ladepfad, wobei der Widerstand von der Überwachungseinheit in den leitenden Zustand gebracht wird, wenn die von der Einheit erfasste Speicherspannung einen vorbestimmten ersten unteren Schwellwert unterschreitet. Vorzugsweise enthält die Steuereinheit einen Mikroprozessor, der über einen ersten Schalter von dem Speicher mit Strom versorgt wird, wobei die Überwachungseinheit so ausgewählt ist, dass sie, wenn die erfasste Speicherspannung einen vorbestimmten zweiten unteren Schwellwert, der vorzugsweise dem ersten unteren Schwellwert entspricht, unterschreitet, den Mikroprozessor durch Öffnen des ersten Schalters von der Stromversorgung durch den Speicher abtrennt. Auf diese Weise kann verhindert werden, dass der Mikroprozessor in einem Unterspannungsbereich arbeitet, in welchem Fehlfunktionen auftreten können. Außerdem kann dadurch die Leistungsentnahme aus dem Speicher verringert werden, was den Zeitraum bis zur vollständigen Entladung des Speichers vergrößert und damit die Gefahr einer vollständigen Entladung verringert.

Vorzugsweise ist die Überwachungseinheit so ausgebildet, dass sie, wenn die erfasste Speicherspannung einen vorbestimmten ersten oberen Schwellwert überschreitet, den Mikroprozessor durch Schließen des ersten Schalters an die Stromversorgung durch den Speicher anschließt und die Steuerung des ersten Schalters an den Mikroprozessor übergibt. Dadurch wird einerseits ein "schlagartiges" Einschalten der Versorgungsspannung und damit eine sehr steile Anstiegsflanke realisiert, was das Anschwingen eines dem Mikroprozessor zugeordneten Oszillators erleichtert. Andererseits kann auf diese Weise eine Hysterese eingestellt werden, so dass der Mikroprozessor erst bei Erreichen einer voll ausreichenden Speicherspannung wieder in Betrieb genommen wird.

Ferner ist die Überwachungseinheit vorzugsweise so ausgebildet, dass sie, wenn die Speicherspannung einen vorbestimmten Maximalwert überschreitet, eine Überladung des Speichers unabhängig von der Steueranordnung dadurch verhindert, dass sie den Ladepfad so schaltet, dass eine weitere Beaufschlagung des Speichers mit Ladestrom verhindert wird.

In weiterer bevorzugter Ausgestaltung ist eine Einrichtung vorgesehen, welche extern betätigbar ist, um den steuerbaren Widerstand mindestens bei vollständig entladenem Energiespeicher zu überbrücken. Die Überbrückungseinrichtung kann beispielsweise von einem magnetisch betätigbaren Schalter oder einer in Sperrrichtung gepolten Diode gebildet werden. Die Überbrückungseinrichtung stellt auch bei völlig entladenem Speicher, wenn auch die Überwachungseinrichtung nicht mehr arbeiten kann, sicher, dass der Speicher wieder mit Ladestrom beaufschlagt werden kann.

Weitere bevorzugte Ausgestaltungen der Erfindung ergeben sich aus den Unteransprüchen.

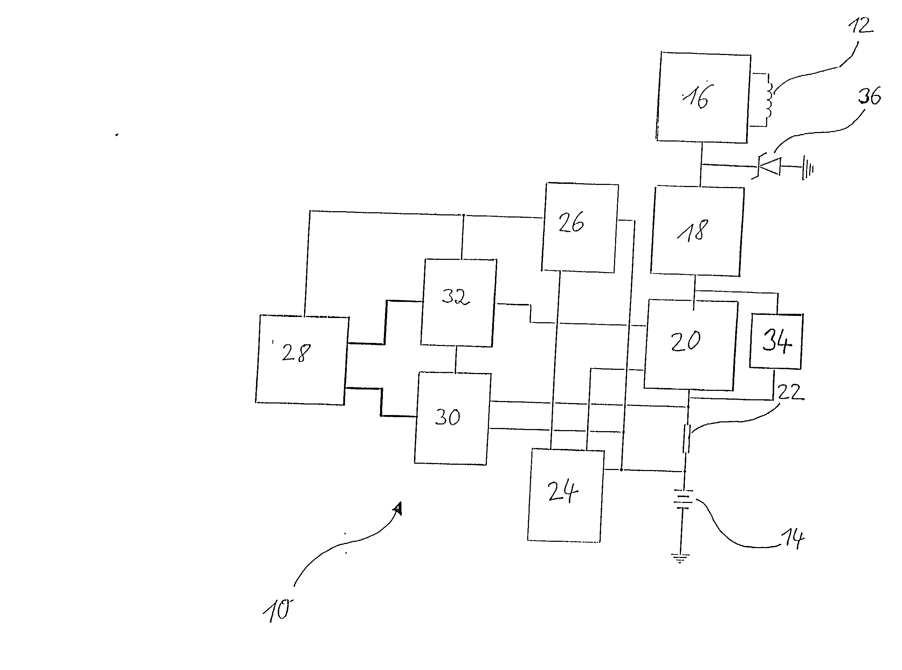

Im folgenden ist eine Ausführungsform der Erfindung anhand der beigefügten Zeichnung beispielhaft näher erläutert, welche ein Blockschaltbild einer erfindungsgemäßen implantierbaren Speicheranordnung für ein medizinisches Implantat zeigt.

Die einzige Figur zeigt schematisch die Verschaltung der wesentlichen Elemente einer implantierbaren Speicheranordnung 10, die als Stromversorgung für ein medizinisches Implantat dient, bei welchem es sich beispielsweise um ein voll implantierbares Hörsystem für die direkte mechanische Stimulation des Mittel- oder Innenohres oder die elektrische Stimulation des Innenohres handeln kann. Eine Ladespule 12 dient dazu, von einem nicht dargestellten externen Ladegerät induktiv transkutan übertragene elektrische Energie aufzunehmen und in den Ladepfad eines wiederaufladbaren Energiespeichers 14, bei dem es sich beispielsweise um eine NiMH-Batterie handeln kann, einzuspeisen. Die in der Ladespule 2 von dem externen Ladegerät induzierte Spannung wird in einer Einheit 16 gleichgerichtet und aufbereitet. Eine Zenerdiode 36 ist vorgesehen, um auf Ladespannungspotential liegende elektronische Bauelemente vor einer zu hohen Ladespannung zu schützen.

Der Ladestrom fließt durch einen im Normalbetrieb geschlossenen mechanischen Schalter 18, ein Regel- und Schaltglied 20, bei dem es sich vorzugsweise im wesentlichen um einem FET handelt und das als steuerbarer Widerstand im Ladestrompfad wirkt, und einen Shuntwiderstand 22 zur Batterie 14. Eine Überwachungseinheit 24 ist direkt mit der Batterie 14 verbunden und dient zur Erfassung und Überwachung der Batteriespannung. Ausgangsseitig ist die Überwachungseinheit 24 einerseits mit dem Regel- und Schaltglied 20 und andererseits mit einem Schalter 26 verbunden, über welchen ein Mikroprozessorsystem 28 mit der Batterie 14 als Spannungsversorgung verbunden ist. Das Mikroprozessorsystem 28 wird vorzugsweise von einem (nicht dargestellten) Pierce-Oszillator getaktet. Ein A/D-Wandler 30 dient dazu, die Spannung der Batterie 14 sowie den Spannungsabfall an dem Shuntwiderstand 22 zu messen, wodurch die beiden wichtigsten Parameter beim Aufladen der Batterie 14, nämlich die Batteriespannung und der Ladestrom, gemessen werden können. Diese Werte werden als Ausgabe des A/D-Wandlers 30 dem Mikroprozessorsystem 28 als Eingangssignal zugeführt, welcher in Abhängigkeit von der erfassten Batteriespannung und dem erfassten Ladestrom über einen D/A-Wandler 32 das Regel/Schaltglied 20 und damit den Ladestrom bzw. die Ladespannung entsprechend einer vorgegebenen Ladestrategie steuert. Insbesondere ist dabei in bekannter Weise eine Überwachungsfunktion implementiert, die dafür sorgt, dass nach Erreichen eines vorgegebenen Ladeendkriteriums der Aufladevorgang beendet wird, indem das Regel/Schaltglied 20 hochohmig geschaltet wird.

Um eine übermäßige Entladung der Batterie 14 im Betrieb zu verhindern, ist die Energiespeicheranordnung 10 in an sich bekannter Weise mit einer Funktion versehen, welche den Implantatträger rechtzeitig vor einer Entladung der Batterie 14 warnt, um ihn zu der Vornahme eines Aufladevorgangs zu animieren. Falls eine Wiederaufladung nicht erfolgt, weil beispielsweise der Implantatträger verhindert ist, kann die Batteriespannung unter die zulässige Untergrenze absinken. Sobald die Überwachungseinheit 24 feststellt, dass die von ihr erfasste Batteriespannung einen vorgegebenen unteren Schwellwert unterschritten hat, schaltet die Überwachungseinheit 24 einerseits durch Öffnen des Schalters 26 den Mikroprozessor 28 ab, indem dessen Spannungsversorgung unterbrochen wird. Dies stellt sicher, dass der Mikroprozessor 28 nicht bei Versorgungsspannungen betrieben wird, die so niedrig sind, dass der Mikroprozessor 28 falsche logische Operationen ausführen oder den Inhalt von flüchtigen Speichern verlieren kann. Dies wird durch entsprechende Wahl der unteren Spannungsgrenze sichergestellt. Andererseits setzt die Überwachungseinheit 24 bei Unterschreiten der Spannungsuntergrenze das Regel/Schaltglied 20 in einen leitenden Zustand, um sicherzustellen, dass eine Aufladung der Batterie 14 auch im Unterspannungsbereich jederzeit möglich ist und keine Blockade des Ladepfads durch eine Fehlfunktion des Mikroprozessors 28 möglich ist. Als zusätzlicher Effekt durch das Abschalten des Mikroprozessors 28 ergibt sich, dass die Stromaufnahme der Elektronik auf die Stromaufnahme der Überwachungseinheit 24 gesenkt wird, die in der Praxis nur einige 100 nA beträgt. Dies hat den positiven Effekt, dass die Batterie 14 weniger belastet wird und dadurch der Zeitraum bis zur vollständigen Entladung der Batterie 14 verlängert wird, was das Risiko einer vollständigen Entladung verringert.

Bei einem im Unterspannungsbereich vorgenommenen Aufladevorgang steigt die Spannung der Batterie 14 allmählich an. Nachdem die Batteriespannung den vorgegebenen unteren Spannungsgrenzwert der Überwachungseinheit 24 um den Wert der Hysterese überschritten hat, schaltet die Überwachungseinheit 24 durch Schließen des Schalters 26 das Mikroprozessorsystem 28 wieder ein, wodurch dieses beginnt, den Aufladevorgang in der oben beschriebenen Weise zu steuern und zu überwachen. Dadurch wird die Steuerung des Regel/Schaltglieds 20 von der Überwachungseinheit 24 an das Mikroprozessorsystem 28 übergeben. Durch das sprunghafte Einschalten der Versorgungsspannung durch das Schließen des Schalters 26 wird ein steiler Spannungsanstieg realisiert, welcher das Anschwingen des Pierce-Oszillators des Mikroprozessorsystems 28 in sicherer Weise gewährleistet.

Ferner ist die Überwachungseinheit 24 so ausgebildet, dass sie, wenn die von ihr erfasste Batteriespannung einen vorbestimmten Maximalwert überschreitet, das Regel/Schaltglied 20 nichtleitend schaltet, um eine Überladung der Batterie 14 selbst dann zu verhindern, wenn das Mikroprozessorsystem 28 aufgrund eines Fehlers den Ladevorgang nach Erreichen des vorgegebenen Ladeendkriteriums nicht beendet.

Der mechanische Schalter 18 in an sich bekannter Weise so ausgebildet, dass er auf eine mechanische Ausdehnung der Batterie 14, wie sie bei der mit einer Überladung einhergehenden übermäßigen Gasentwicklung auftritt, anspricht und den Ladepfad unterbricht oder den Empfang von Ladeenergie unmöglich macht, um ein weiteres Aufladen der Batterie 14 zu verhindern.

Auf diese Weise sind bei der beschriebenen Speicheranordnung 10 drei unabhängige Überwachungskreise realisiert, welche die Aufladung der Batterie rechtzeitig beenden, um Schäden zu vermeiden. Dabei handelt es sich primär um das Mikroprozessorsystem 28, welches den Ladevorgang bei Erreichen eines vorgegebenen Ladeendkriteriums beendet. Unabhängig davon beendet die Überwachungseinheit 24 den Ladevorgang, wenn die von ihr unabhängig von dem Mikroprozessorsystem 28 erfasste Batteriespannung einen vorgegebenen Maximalwert überschreitet. Schließlich beendet der mechanische Schalter 18 bei Versagen der ersten beiden Überwachungskreise elektronikunabhängig den Ladevorgang so rechtzeitig, dass eine Beschädigung der Batterie 14 und eine Gefährdung des Implantatträgers zuverlässig verhindert wird.

Die Überwachungseinheit 24 sorgt ferner dafür, dass unabhängig von dem Mikroprozessorsystem 28 eine drohende übermäßige Entladung der Batterie 14 erkannt wird und möglicherweise daraus resultierende Fehlfunktionen des Mikroprozessorsystems 28 durch Abschalten desselben verhindert werden. Ferner übernimmt die Überwachungseinheit 24 in diesem Fall die Steuerung des Ladepfads, wobei jederzeit unabhängig von dem Mikroprozessorsystem 28 im Unterspannungsbereich sichergestellt wird, dass der Ladepfad leitend ist, so dass jederzeit eine Wiederaufladung der Batterie 14 möglich ist. Durch das schlagartige Wiedereinschalten der Versorgungsspannung des Mikroprozessorsystems 28 durch Schließen des Schalters 26 durch die Überwachungseinheit 24 können ferner Anlaufprobleme des Pierce-Oszillators des Mikroprozessorsystems 28 verhindert werden.

Parallel zu dem Regel/Schaltglied 20 ist eine Überbrückungseinrichtung 34 geschaltet, die extern, d.h. von außerhalb des Körpers betätigbar ist, um das Regel/Schaltglied 20 zu überbrücken bzw. kurzzuschließen. Die Überbrückungseinrichtung 34 ist für den Fall vorgesehen, wenn der Speicher 14 soweit entladen ist, dass die Speicherspannung so weit abgefallen ist, dass sie für eine ordnungsgemäße Funktion der Überwachungseinheit 24 nicht mehr ausreichend ist. Dabei kann der Fall eintreten, dass sich das Regel/Schaltglied 20 in einen Zustand mit so hohem Widerstand kommt, der ein Aufladen des Speichers 14 über den normalen Ladepfad nicht ermöglicht. In diesem Fall kann durch Betätigung der Überbrückungseinrichtung 34 von außen eingegriffen werden, um das Regel/Schaltglied 20 zu überbrücken und so ein sicheres Aufladen des Speichers 20 auch bei extremer Unterspannung oder vollständiger Entladung zu ermöglichen.

Die Überbrückungseinrichtung 34 kann beispielsweise von einem mittels eines externen Magneten schließbaren mechanischen Schalter gebildet werden (Reed-Schalter), wobei der Magnet vorteilhafterweise in das externe Ladegerät integriert sein kann. In diesem Fall wird, wenn das Ladegerät an die Haut des Implantatträgers gehalten wird, der Schalter 34 geschlossen und ein Aufladen ist über den über den Schalter 34 führenden Strompfad möglich. Sobald jedoch die Speicherspannung für einen sicheren Betrieb der Überwachungseinheit 24 wieder ausreicht, sollte der Schalter 34 durch Entfernen des Magneten wieder geöffnet werden, um die oben beschriebene normale Ladefunktion wieder zu aktivieren und insbesondere ein Überladen zu verhindern.

In alternativer Ausgestaltung kann die Überbrückungseinrichtung 34 auch von einer in Sperrrichtung gepolten Diode, beispielsweise einer Zenerdiode, gebildet werden. Die Sperrspannung der Diode ist dabei so bemessen, dass sie oberhalb der im normalen Lademodus auftretenden Ladespannungen liegt und so den normalen Ladevorgang nicht beeinflusst, jedoch unterhalb der Sperrspannung der Schutzdiode 36 liegt. Im Notfall, d.h. wenn die Speicherspannung nicht mehr zum Betrieb der Überwachungseinheit 24 ausreicht, wird dann zum Beginn des Wiederaufladens ein spezielles externes Notladegerät verwendet, welches sich von dem für den normalen Ladevorgang verwendeten externen Ladegerät im wesentlichen dadurch unterscheidet, dass es dafür sorgt. dass eine Ladespannung erzeugt wird, die wesentliche höher als beim normalen Ladevorgang ist und oberhalb der Sperrspannung der Überbrückungsdiode 34, jedoch unterhalb der Sperrspannung der Schutzdiode 36 liegt. Auf diese Weise wird mit dem Notladegerät eine Überbrückung des Regel/Schaltglieds20 erreicht. Statt der Verwendung eines speziellen Notladegeräts kann auch des normale Ladegerät mit einem umschaltbaren Notlademodus versehen sein. Jedoch sollte bei Erreichen einer Speicherspannung, die für einen sicheren Betrieb der Überwachungseinheit 24 wieder ausreicht, der Notlademodus beendet werden, um die Überbrückungsdiode 34 wieder sperrend zu machen und somit die normale Ladefunktion wieder zu aktivieren und insbesondere ein Überladen zu verhindern.

Mittels der Überbrückungseinheit 34 wird somit sichergestellt, dass selbst bei vollständig entladenem Speicher 14 durch externe Betätigung ein Wiederaufladen möglich ist.

## Patentansprüche

1. Implantierbare Energiespeicheranordnung für ein medizinisches Implantat, mit einem wiederaufladbaren Speicher (14) für elektrische Energie, sowie einer Einheit (20, 22, 26, 28, 30, 32) zum Steuern des Ladevorgangs, **dadurch gekennzeichnet, dass** eine von der Steuereinheit (20, 22, 26, 28, 30, 32) unabhängige Überwachungseinheit (24) vorgesehen ist, welche die Speicherspannung unabhängig von der Steuereinheit erfasst und so ausgebildet ist, dass sie, wenn die von ihr erfasste Speicherspannung außerhalb eines vorgegebenen Bereichs liegt, die Steuerung des Ladepfads übernimmt.

2. Speicheranordnung nach Anspruch 1, **dadurch gekennzeichnet, dass** die Überwachungseinheit (24) so ausgebildet ist, dass sie, wenn die von ihr erfasste Speicherspannung einen vorbestimmten ersten unteren Schwellwert unterschreitet, dafür sorgt, dass der Ladepfad so geschaltet ist, dass der Speicher (14) mit einem Ladestrom beaufschlagbar ist.

3. Speicheranordnung nach Anspruch 2, **dadurch gekennzeichnet, dass** die Steuereinheit (20, 22, 26, 28, 30, 32) den Ladevorgang über ein Stellglied (20) im Ladepfad steuert, wobei das Stellglied von der Überwachungseinheit (24) in den leitenden Zustand gebracht wird, wenn die von der Überwachungseinheit erfasste Speicherspannung den vorbestimmten ersten unteren Schwellwert unterschreitet.

4. Speicheranordnung nach Anspruch 3, **dadurch gekennzeichnet, dass** das Stellglied (20) von einem steuerbaren Widerstand gebildet wird.

5. Speicheranordnung nach Anspruch 4, **dadurch gekennzeichnet, dass** die Steuereinheit (20, 22, 26, 28, 30, 32) ein Mikroprozessorsystem (28) enthält, das über einen ersten Schalter (26) von dem Speicher (14) mit Spannung versorgt wird, wobei die Überwachungseinheit (24) so ausgebildet ist, dass sie, wenn die erfasste Speicherspannung einen vorbestimmten zweiten unteren Schwellwert unterschreitet, das Mikroprozessorsystem durch Öffnen des zweiten Schalters von der Spannungsversorgung durch den Speicher abtrennt.

6. Speicheranordnung nach Anspruch 5, **dadurch gekennzeichnet, dass** der erste und der zweite untere Schwellwert identisch sind.

7. Speicheranordnung nach Anspruch 5 oder 6, **dadurch gekennzeichnet, dass** die Überwachungseinheit (24) so ausgebildet ist, dass sie, wenn die erfasste Speicherspannung einen vorbestimmten ersten oberen Schwellwert überschreitet, das Mikroprozessorsystem (28) durch Schließen des ersten Schalters (26) an die Spannungsversorgung durch den Speicher (14) anschließt und die Steuerung des Stellglieds (20) an das Mikroprozessorsystem übergibt.

8. Speicheranordnung nach einem der Ansprüche 5 bis 7, **dadurch gekennzeichnet, dass** das Mikroprozessorsystem (28) von einem Pierce-Oszillator getaktet wird.

9. Speicheranordnung nach einem der Ansprüche 3 bis 8, **dadurch gekennzeichnet, dass** die Steuereinheit (20, 22, 26, 28, 30, 32) eine Einrichtung (30) zum Erfassen der Speicherspannung und des Stroms im Ladestromkreis umfasst und das Stellglied (20) gemäß einem Ladeprogramm in Abhängigkeit von dem so erfassten Werten steuert.

10. Speicheranordnung nach einem der vorhergehenden Ansprüche, **dadurch gekennzeichnet, dass** die Überwachungseinheit (24) so ausgebildet ist, dass sie, wenn die Speicherspannung einen vorbestimmten Maximalwert überschreitet, den Ladepfad so schaltet, dass eine weitere Beaufschlagung des Speichers (14) mit Ladestrom verhindert wird.

11. Speicheranordnung nach Anspruch 10, sofern auf Anspruch 3 rückbezogen, **dadurch gekennzeichnet, dass** die Überwachungseinheit (24) so ausgebildet ist, dass sie, wenn die Speicherspannung den vorbestimmten Maximalwert überschreitet, das Stellglied (20) nichtleitend schaltet.

12. Speicheranordnung nach Anspruch 5, **dadurch gekennzeichnet, dass** ein zweiter Schalter (18) vorgesehen ist, der auf mechanische Veränderungen des Speichers (14) durch Überladung anspricht und dabei den Speicher von der Ladestromquelle abkoppelt oder den Empfang von Ladeenergie verhindert.

13. Speicheranordnung nach Anspruch 12, **dadurch gekennzeichnet, dass** der zweite Schalter (18) auf eine Ausdehnung des Speichers (14) anspricht und dabei den Ladestrompfad unterbricht oder den Empfang von Ladeenergie verhindert.

14. Speicheranordnung nach einem der vorhergehenden Ansprüche, **dadurch gekennzeichnet, dass** eine Einrichtung (12, 16) vorgesehen ist, um Ladeenergie, die induktiv von einer externen Quelle transkutan übertragen wird, aufzunehmen und für eine Einspeisung in den Ladestrompfad aufzubereiten.

15. Speicheranordnung nach einem der vorhergehenden Ansprüche, **dadurch gekennzeichnet, dass** es sich bei dem medizinischen Implantat um ein vollimplantierbares Hörsystem handelt.

16. Speicheranordnung nach Anspruch 4 oder einem der darauf rückbezogenen Ansprüche, **dadurch gekennzeichnet, dass** der steuerbare Widerstand (20) von einem FET gebildet wird.

17. Speicheranordnung nach Anspruch 4 oder einem der darauf rückbezogenen Ansprüche, **dadurch gekennzeichnet, dass** eine Einrichtung vorgesehen ist, welche extern betätigbar ist, um den steuerbaren Widerstand mindestens bei vollständig entladenem Energiespeicher zu überbrücken.

18. Speicheranordnung nach Anspruch 17, **dadurch gekennzeichnet, dass** es sich bei der Überbrückungseinrichtung um einen magnetisch betätigbaren Schalter handelt.

19. Speicheranordnung nach Anspruch 17, **dadurch gekennzeichnet, dass** es sich bei der Überbrückungseinrichtung um eine in Sperrrichtung gepolte Diode handelt.

20. Speicheranordnung nach Anspruch 18, **dadurch gekennzeichnet, dass** eine Schutzdiode für die Steuereinheit und die Überwachungseinrichtung vorgesehen ist, wobei die Sperrspannung der Schutzdiode größer als diejenige der Überbrückungsdiode ist.

21. Verfahren zum Betreiben einer implantierbaren Energiespeicheranordnung für ein medizinisches Implantat, mit einem wiederaufladbaren Speicher (14) für elektrische Energie, wobei im Normalbetrieb der Ladevorgang mittels einer Steuereinheit (20, 22, 26, 28, 30, 32) gesteuert wird, **dadurch gekennzeichnet, dass** mittels einer von der Steuereinheit (20, 22, 26, 28, 30, 32) unabhängigen Überwachungseinheit (24) die Speicherspannung unabhängig von der Steuereinheit erfasst wird, wobei die Überwachungseinheit, wenn die von ihr erfasste Speicherspannung außerhalb eines vorgegebenen Bereichs liegt, die Steuerung des Ladepfads übernimmt.

## Claims

1. An implantable energy storage system for a medical implant, comprising a rechargeable storage (14) for electric energy and a unit (20, 22, 26, 28, 30, 32) for controlling the charging process, **characterized in that** a monitoring unit (24) which is independent of the control unit (20, 22, 26, 28, 30, 32) is provided for sensing storage voltage independently of the control unit and is configured such that it assumes control of a charging path when the storage voltage sensed by it lies outside a predetermined range.

2. The storage system as claimed in claim 1, **characterized in that** the monitoring unit (24) is configured to ensure switching of the charging path such that a charging current can be supplied to the storage (14) when the storage voltage sensed by it falls below a predetermined first lower threshold.

3. The storage system as claimed in claim 2, **characterized in that** the control unit (20, 22, 26, 28, 30, 32) controls the charging process via an actuator (20) in the charging path, the actuator being made electrically conductive by the monitoring unit (24) when the storage voltage sensed by the monitoring unit falls below the predetermined first lower threshold.

4. The storage system as claimed in claim 3, **characterized in that** the actuator (20) is formed by a controllable resistor.

5. The storage system as claimed in claim 4, **characterized in that** the control unit (20, 22, 26, 28, 30, 32) comprises a microprocessor system (28) which is supplied with voltage by the storage (14) via a first switch (26), wherein the monitoring unit (24) is configured to cut off the microprocessor system from voltage supply by the storage by opening a second switch when the sensed storage voltage falls below a predetermined second lower threshold.

6. The storage system as claimed in claim 5, **characterized in that** the first lower threshold and the second lower threshold are identical.

7. The storage system as claimed in claim 5 or 6, **characterized in that** the monitoring unit (24) is configured to connect the microprocessor system (28), by closing the first switch (26), to the voltage supply by the storage (14) and to transfer control of the actuator (20) to the microprocessor system when the sensed storage voltage exceeds a predetermined first upper threshold.

8. The storage system as claimed in one of claims 5 to 7, **characterized in that** a Pierce oscillator sends clock signals to the microprocessor system (28).

9. The storage system as claimed in one of claims 3 to 8, **characterized in that** the control unit (20, 22, 26, 28, 30, 32) comprises a means (30) for sensing the storage voltage and the current in a charging circuit, and for controlling an actuator (20) according to a charging program as a function of the values sensed **in that** way.

10. The storage system as claimed in one of the preceding claims, **characterized in that** the monitoring unit (24) is configured to switch the charging path such that charging current is prevented from further flowing to the storage (14) when the storage voltage exceeds a predetermined maximum value.

11. The storage system as claimed in claim 10, if referred back to claim 3, **characterized in that** the monitoring unit (24) is configured to make the actuator (20) non-conductive when the storage voltage exceeds the predetermined maximum voltage.

12. The storage system as claimed in claim 5, **characterized in that** a second switch (18) is provided which is responsive to mechanical changes of the storage (14) caused by overcharging and either decouples the storage from a charging current source or prevents reception of charging energy.

13. The storage system as claimed in claim 12, **characterized in that** the second switch (18) is responsive to an expansion of the storage (14) and thereby interrupts the charging current path or prevents reception of charging energy.

14. The storage system as claimed in one of the preceding claims, **characterized in that** a means (12, 16) is provided for picking up charging energy which is inductively transmitted transcutaneously from an external source and for conditioning the charging energy to be fed into the charging current path.

15. The storage system as claimed in one of the preceding claims, **characterized in that** the medical implant is a fully implantable hearing system.

16. The storage system as claimed in claim 4 or in a claim referred back thereto, **characterized in that** the controllable resistor (20) is formed by a FET.

17. The storage system as claimed in claim 4 or in claims referred back thereto, **characterized in that** a means which can be externally activated is provided for bypassing the controllable resistor at least when the energy storage has been completely discharged.

18. The storage system as claimed in claim 17, **characterized in that** the bypass means is a magnetically actuatable switch.

19. The storage system as claimed in claim 17, **characterized in that** the bypass means is a diode poled in a reverse direction.

20. The storage system as claimed in claim 18, **characterized in that** a protective diode is provided for the control unit and the monitoring means, the protective diode having a reverse voltage which is greater than a reverse voltage of the bypass diode.

21. A method for operating an implantable energy storage system for a medical implant, with a rechargeable storage (14) for electric energy, wherein during normal operation the charging process is controlled by means of a control unit (20, 22, 26, 28, 30, 32), **characterized in that** with the help of a monitoring unit (24) which is independent of the control unit (20, 22, 26, 28, 30, 32) the storage voltage is sensed independently of the control unit, wherein the monitoring unit assumes control of the charging path when the storage voltage sensed by it lies outside a predetermined range.

## Revendications

1. Dispositif accumulateur d'énergie implantable pour un implant médical, comportant un accumulateur rechargeable (14) pour de l'énergie électrique ainsi qu'une unité (20, 22, 26, 28, 30, 32) de commande du processus de charge, **caractérisé en ce qu'**est pourvue une unité de surveillance (24) indépendante de l'unité de commande (20, 22, 26, 28, 30, 32) qui enregistre la tension d'accumulateur indépendamment de l'unité de commande, et qui est constituée de telle sorte qu'elle prend en charge la commande du circuit de charge quand la tension d'accumulateur qu'elle enregistre sort d'une plage prédéterminée.

2. Dispositif accumulateur selon la revendication 1, **caractérisé en ce que** l'unité de surveillance (24) est constituée de telle sorte que, quand la tension d'accumulateur qu'elle enregistre passe sous une première valeur seuil inférieure prédéterminée, elle assure que le circuit de charge est commuté pour que l'accumulateur (14) puisse recevoir un courant de charge.

3. Dispositif accumulateur selon la revendication 2, **caractérisé en ce que** l'unité de commande (20, 22, 26, 28, 30, 32) commande le processus de charge via un actionneur (20) dans le circuit de charge, dans lequel l'actionneur est amené à l'état conducteur par l'unité de surveillance (24) quand la tension d'accumulateur enregistrée par l'unité de surveillance passe sous la première valeur seuil inférieure prédéterminée.

4. Dispositif accumulateur selon la revendication 3, **caractérisé en ce que** l'actionneur (20) est constitué à partir d'une résistance réglable.

5. Dispositif accumulateur selon la revendication 4, **caractérisé en ce que** l'unité de commande (20, 22, 26, 28, 30, 32) comporte un système à microprocesseur (28) qui est alimenté en tension par l'accumulateur (14) via un premier interrupteur (26), dans lequel l'unité de surveillance (24) est constituée de telle sorte qu'elle coupe le système à microprocesseur de l'alimentation de tension par l'accumulateur en ouvrant le deuxième interrupteur quand la tension d'accumulateur enregistrée passe sous une deuxième valeur seuil inférieure prédéterminée.

6. Dispositif accumulateur selon la revendication 5, **caractérisé en ce que** les première et deuxième valeurs seuil inférieures sont identiques.

7. Dispositif accumulateur selon la revendication 5 ou 6, **caractérisé en ce que** l'unité de surveillance (24) est constituée de telle sorte que, quand la tension d'accumulateur enregistrée dépasse une première valeur seuil supérieure prédéterminée, elle connecte le système à microprocesseur (28) à l'alimentation de tension par l'accumulateur (14) en fermant le premier interrupteur (26) et transfère la commande de l'actionneur (20) au système à microprocesseur.

8. Dispositif accumulateur selon l'une des revendications 5 à 7, **caractérisé en ce que** le système à microprocesseur (28) est synchronisé par un oscillateur Pierce.

9. Dispositif accumulateur selon l'une des revendications 3 à 8, **caractérisé en ce que** l'unité de commande (20, 22, 26, 28, 30, 32) comporte un dispositif (30) pour enregistrer la tension d'accumulateur et le courant dans le circuit de courant de charge, et **en ce que** l'actionneur (20) commande selon un programme de charge en fonction des valeurs ainsi enregistrées.

10. Dispositif accumulateur selon l'une des revendications précédentes, **caractérisé en ce que** l'unité de surveillance (24) est constituée de telle sorte que, quand la tension d'accumulateur dépasse une valeur maximale prédéterminée, elle commute le circuit de charge de manière à ce que l'accumulateur (14) ne puisse plus recevoir un courant de charge.

11. Dispositif accumulateur selon la revendication 10, en référence à la revendication 3, **caractérisé en ce que** l'unité de surveillance (24) est constituée de telle sorte que, quand la tension de l'accumulateur dépasse la valeur maximale prédéterminée, elle commute l'actionneur (20) pour qu'il ne conduise plus.

12. Dispositif accumulateur selon la revendication 5, **caractérisé en ce qu'**est pourvu un deuxième interrupteur (18) qui est actionné lors de modifications mécaniques de l'accumulateur (14) par une surcharge et découple ainsi l'accumulateur de la source de courant de charge ou empêche la réception d'énergie de charge.

13. Dispositif accumulateur selon la revendication 12, **caractérisé en ce que** le deuxième interrupteur (18) est actionné par une dilatation de l'accumulateur (14) et interrompt ainsi le circuit du courant de charge ou empêche la réception d'énergie de charge.

14. Dispositif accumulateur selon l'une des revendications précédentes, **caractérisé en ce qu'**un dispositif (12, 16) est pourvu pour recevoir de l'énergie de charge qui est transmise par induction de manière transcutanée à partir d'une source externe et la transformer pour une alimentation dans le circuit de courant de charge.

15. Dispositif accumulateur selon l'une des revendications précédentes, **caractérisé en ce que** l'implant médical est un système auditif entièrement implantable.

16. Dispositif accumulateur selon la revendication 4 ou l'une des revendications qui s'y réfère, **caractérisé en ce que** la résistance réglable (20) est constituée à partir d'un transistor FET.

17. Dispositif accumulateur selon la revendication 4 ou l'une des revendications qui s'y réfèrent, **caractérisé en ce qu'**est pourvu un dispositif à commande externe pour ponter la résistance réglable au moins lorsque l'accumulateur d'énergie est entièrement déchargé.

18. Dispositif accumulateur selon la revendication 17, **caractérisé en ce que** le dispositif de pontage est un interrupteur à actionnement magnétique.

19. Dispositif accumulateur selon la revendication 17, **caractérisé en ce que** le dispositif de pontage est une diode branchée en direction bloquante.

20. Dispositif accumulateur selon la revendication 18, **caractérisé en ce qu'**une diode de protection est pourvue pour l'unité de commande et le dispositif de surveillance, dans lequel la tension bloquante de la diode de protection est supérieure à celle de la diode de pontage.

21. Procédé d'utilisation d'un dispositif accumulateur d'énergie implantable pour un implant médical comportant un accumulateur rechargeable (14) pour de l'énergie électrique, dans lequel le processus de charge est commandé en fonctionnement normal par une unité de commande (20, 22, 26, 28, 30, 32), **caractérisé en ce que**, à l'aide d'une unité de surveillance (24) indépendante de l'unité de commande (20, 22, 26, 28, 30, 32), la tension d'accumulateur est enregistrée indépendamment de l'unité de surveillance, dans lequel l'unité de surveillance prend en charge la commande du circuit de charge quand la tension d'accumulateur qu'elle enregistre sort d'une plage prédéterminée.
